(19) Europäisches Patentamt
European Patent Office
Office européen des brevets

(11) Veröffentlichungsnummer: **0 207 252 B1**

(12) # EUROPÄISCHE PATENTSCHRIFT

(45) Veröffentlichungstag der Patentschrift: **29.07.92**

(51) Int. Cl.$^5$: **C12Q 1/34**, C07C 69/00

(21) Anmeldenummer: **86106022.6**

(22) Anmeldetag: **02.05.86**

(54) **Aromatische Hydroxy- oder Thiol-Derivate von Carbonsäureestern als Lipasesubstrate.**

(30) Priorität: **03.05.85 DE 3516001**

(43) Veröffentlichungstag der Anmeldung:
**07.01.87 Patentblatt 87/02**

(45) Bekanntmachung des Hinweises auf die Patenterteilung:
**29.07.92 Patentblatt 92/31**

(84) Benannte Vertragsstaaten:
**AT BE CH DE FR GB IT LI LU NL SE**

(56) Entgegenhaltungen:
**EP-A- 0 115 394**
**EP-A- 0 137 177**
**DE-B- 2 904 305**
**FR-A- 2 022 180**
**FR-A- 2 273 283**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Neumann, Ulrich, Dr. rer. nat.**
**Drosselweg 2**
**W-8132 Peissenberg/Obb.(DE)**
Erfinder: **Junius, Martina Dr. rer. nat.**
**Eichstrasse 4**
**W-8139 Bernried(DE)**
Erfinder: **Batz, Hans-Georg, Dr. rer. nat.**
**Traubinger Strasse 63**
**W-8132 Tutzing(DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86(DE)**

**EP 0 207 252 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Lipase (Triacylglycerinacylhydrolase EC 3.1.1.3) hydrolysiert emulgierte Triglyceride langkettiger Fettsäuren an der Grenzfläche zwischen Öltröpfchen und wäßriger Phase. Bei bestimmten Erkrankungen, wie akuter Pankreatitis oder Pankreaskarzinom wird die normalerweise sehr niedrige Lipasekonzentration im Serum erhöht und die Bestimmung der Lipaseaktivität hat daher diagnostisch beträchtliche Bedeutung. Die Lipasebestimmung ist daher für die klinische Chemie, aber auch für die Biochemie, die pharmazeutische Chemie und die Lebensmittelchemie von erheblicher Bedeutung.

Es sind bereits eine Reihe von Lipasemeßmethoden bekannt. So wird titrimetrisch mit Lauge die freigesetzte Säure bestimmt. Die Methode ist störanfällig und nicht besonders spezifisch. Weiter ist bekannt die photometrische Bestimmung im UV. So wird in der DE-OS 33 42 106 die Verwendung eines Mono- oder Diglycerids einer höheren Fettsäure in Kombination mit einem nichtionischen Tensid als Substrat für die UV-Bestimmung beschrieben. UV-Tests benötigen jedoch eine relativ aufwendige Meßapparatur und lassen mögliche Störungen wie Gerätedefekte, Erschöpfung des Reagenz, oft nicht ohne weiteres erkennen. Daher besteht Bedarf an einem Farbtest, der mit einer einfachen Apparatur durchgeführt und direkt visuell kontrolliert werden kann.

Bekannt ist bereits der Farbtest von Kurooka unter Verwendung eines Dimerkaptopropanol-Triesters als Substrat zusammen mit einer Dithiobisnitrobenzoesäure als Chromogen, einem Esteraseninhibitor und einem Lipasenaktivator.

Dieses Verfahren weist jedoch eine unbefriedigende Genauigkeit auf (J. Clin. Chem. Clin. Biochem. 20, 537-552 (1982)). Weiter ist bekannt ein Verfahren mit Trilinolein als Substrat, Lipoxygenase als Hilfssystem, wobei in einer anschließenden Farbreaktion durch Fettsäurehydroperoxid Eisen-II zu Eisen-III oxydiert und als Eisen-III-thiocyanat nachgewiesen wird. Diese Methode ergibt keine zuverlässigen Ergebnisse (J. Clin. Chem. Biochem. 20, 745-752 (1982)).

Bekannt ist auch eine Trübungsbestimmung, die jedoch eine relativ geringe Empfindlichkeit aufweist.

Der Erfindung liegt daher die Aufgabe zugrunde, einen Farbtest zur Bestimmung der Lipase zu schaffen, der die Nachteile der bekannten Farbtests nicht aufweist, genaue Ergebnisse liefert, einfach in der Handhabung ist, hohe Empfindlichkeit besitzt und nur eine geringe lag-Phase aufweist, so daß die Adaptation an die verschiedenen Analysenautomatensysteme unschwierig ist.

Gelöst wird diese Aufgabe erfindungsgemäß durch ein Lipasesubstrat der allgemeinen Formel

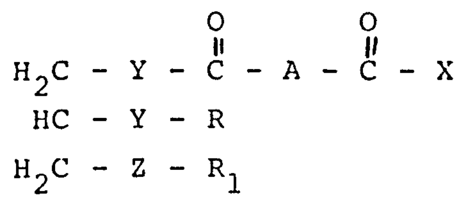

worin
A eine Alkylen- oder Alkenylengruppe mit 1 bis 16 C-Atomen,
R und $R_1$ die gleich oder verschieden sein können, je eine Alkyl-, Alkenyl- oder Acylgruppe mit 1 bis 20 C-Atomen oder eine gegebenenfalls alkylsubstituierte Aryl- oder Aralkylgruppe mit 1 bis 8 C-Atomen in Alkylrest, einer der Reste R und $R_1$ auch ein Wasserstoffatom,
X den Rest einer aromatischen Hydroxy- oder Thiolverbindung und jedes Y und Z unabhängig voneinander -S-oder -O-, Z auch $-CH_2-$, bedeuten.

Unter der Einwirkung der Lipase wird das erfindungsgemäße Lipasesubstrat gespalten unter Freisetzung der dem Rest X entsprechenden aromatischen Hydroxy- oder Thiolverbindung, die man entweder direkt optisch bestimmt oder mit einem geeigneten Chromophor oder Fluorophor kuppelt und das Kupplungsprodukt mißt.

R oder/und $R_1$ weisen vorzugsweise 6 bis 18 C-Atome, besonders bevorzugt 8 bis 12 C-Atome auf. Aufgrund ihrer Hydrolyseunempfindlichkeit werden dabei für R und $R_1$ die Alkylgruppen gegenüber den Acylgruppen besonders bevorzugt. Überraschenderweise erwiesen sich die Verbindungen mit R und $R_1$ = Alkyl, Alkenyl oder Aralkyl als gute Lipasesubstrate, obwohl die natürlichen Triglyceride Acylgruppen tragen.

Beispiele für R oder/und $R_1$ sind Methyl-, Ethyl-, Propyl-, Butyl-, Pentyl-, Hexyl-, Heptyl-, Octyl-, Nonyl-, Decyl-, Undecyl, Dodecyl-, Tetradecyl-, Hexadecyl- und Octadecylreste als Alkylgruppen sowie die entsprechenden Acylgruppen wie die Acetyl-, Propionyl-, Butyryl-, Valeryl-, Capronyl-, Capryl-, Caprinyl-, Lauryl-,

Myristyl-, Palmityl- und Stearylgruppe, die Oleyl-, Crotonyl-, Linolylgruppe, Phenyl-, Benzyl- oder Octylphenylgruppe.

Das erfindungsgemäße Lipasesubstrat enthält weiter den Rest einer Dicarbonsäure COOH-A-COOH, in der A vorzugsweise 3 bis 7 C-Atome aufweist. Beispiele für Säuren, von denen sich A ableitet, sind Malonsäure, Bernsteinsäure, Glutarsäure, Adipinsäure, Pimelinsäure, Korksäure, Azelainsäure, Sebacinsäure, Nonandicarbonsäure, Decandicarbonsäure und Undecandicarbonsäure. Die Säuren von Glutarsäure bis Azelainsäure, die A mit 3 bis 7 C-Atomen entsprechen, werden wie oben erwähnt, bevorzugt.

X kann eine aromatische Hydroxy- oder Thiolverbindung sein, die ein Chromophor darstellt oder erst durch eine Folgereaktion in einen Farbstoff überführt werden kann. Typische Beispiele sind Phenol, Thiophenol, Naphthol, Thionaphthol und deren Derivate sowie die an sich chromogenen Verbindungen wie der Resorufin-, Chlorphenolrot-, Indoxyl- oder Thiofluoreszeinrest. Eine erschöpfende Aufzählung der geeigneten Hydroxy- oder Thiolverbindungen ist aufgrund ihrer großen Zahl nicht möglich, die direkt chromophoren oder in Chromophore überführbaren aromatischen Hydroxy- oder Thiolverbindungen sind dem Fachmann jedoch bekannt.

Bevorzugt sind Chromophore, die eine geringe Polarität aufweisen und lipophil sind. Dabei sollte jedoch die Wasserlöslichkeit noch gewährleistet sein.

Der lipophile Charakter der o.g. Chromophore kann durch geeignete Substitution, wie z.B. mit Alkylgruppen, positiv beeinflußt werden. Als geeignete Substituenten für den Resorufinrest haben sich u.a. die Methyl-, Dimethyl- und Äthylgruppe sowie die Substitution mit Brom als geeignet erwiesen.

Die erfindungsgemäßen Verbindungen sind neu. Sie besitzen ein Asymmetriezentrum und sind daher optisch aktiv. Als Lipasesubstrat können sowohl die bei den üblichen Herstellungsmethoden anfallenden Racemate als auch die optischen Isomeren verwendet werden.

Die Herstellung der erfindungsgemäßen Lipasesubstrate kann nach an sich bekannten Methoden erfolgen. So finden sich geeignete Verfahren zur Synthese der 1.2-O-Diether- und 1.2-Diacylglycerinverbindungen beispielsweise beschrieben in Methods in Enzymology, 98, 623 (1983) und Oleagineux 23, 185 (1968). Die Synthese von Alkandiolderivaten ist beispielsweise in Can. J. Biochem. 46, 69 (1968) beschrieben.

Aus den 1.2-O-Diether- und 1.2-Diacyl-glycerinverbindungen werden dann durch Umsetzung mit den entsprechenden Dicarbonsäureanhydriden in wasserfreiem Medium wie Chloroform/Pyridin die entsprechenden Glycerodicarbonsäure-Monoester erhalten. Geeignete Methoden zur Herstellung der Dicarbonsäureanhydride sind in Houben-Weyl-Müller "Methoden der organischen Chemie", Band IV/4, Seite 786 beschrieben.

Die Veresterung des Monoesters mit der aromatischen Hydroxy- oder Thiolverbindung, von der sich der Rest X ableitet, kann beispielsweise durch direkte Umsetzung des Dicarbonsäuremonoesters mit dem aromatischen Alkohol oder Thiol in Gegenwart eines Wasser entziehenden Mittels wie Dicyclohexylcarbodiimid, durchgeführt werden. Alternativ wird der Dicarbonsäuremonoester zuerst in einen aktivierten Ester überführt, beispielsweise in den Hydroxysuccinimidester oder das Imidazolid und der aktivierte Ester wird dann mit dem aromatischen Alkohol oder Thiol umgesetzt.

Ebenso ist es auch möglich, zuerst einen Monoester der Dicarbonsäure mit dem aromatischen Alkohol oder Thiol herzustellen, beispielsweise den Adipinsäure-Mononitrophenylester oder Glutarsäuremonophenylester, und diesen dann mit dem 1.2-O-Dialkyl- bzw. -Diacylglycerin zu verestern, beispielsweise über intermediäre Bildung eines Säurechlorids, -anhydrids oder aktivierten Esters. Die Herstellung der Dicarbonsäuremonoester mit dem aromatischen Alkohol oder Thiol kann beispielsweise aus Säureanhydrid und aromatischer Verbindung im Molverhältnis 1:1 oder aus Dicarbonsäure und aromatischer Verbindung im Molverhältnis 2:1 oder aus einem Dicarbonsäuremonoester mit leicht abspaltbarer Schutzgruppe und der aromatischen Verbindung erfolgen. Eine geeignete Methode ist beispielsweise in Arch. Pharm. 287, 514 (1954) beschrieben.

Alternativ kann der 1.2-O-Dialkyl- bzw. 1.2-O-Diacylglycero-dicarbonsäure-monoester auch hergestellt werden, indem man zunächst einen Dicarbonsäuremonoester aus der Dicarbonsäure und einem leicht abspaltbaren Alkohol, wie z. B. Benzylalkohol oder 2,2,2-Trichlorethylalkohol herstellt und die so erhaltene Säure dann mit dem erwähnten Dialkyl- bzw. Diacyl-Glycerin verestert. Anschließend wird die Schutzgruppe entfernt und die Umsetzung mit dem aromatischen Alkohol oder Thiol, wie oben beschrieben, vorgenommen.

Eine weitere Herstellmethode besteht darin, daß man zuerst ein geschütztes Glycerin wie 1,2-Isopropylidenglycerin mit einem Dicarbonsäuremonoester umsetzt unter Bildung des entsprechenden geschützten Glycero-3-dicarbonsäure-diesters, dann die Schutzgruppe des Glycerins entfernt und die freigewordenen OH-Gruppen alkyliert bzw. acyliert. Schließlich wird die ursprünglich vorhandene Monoestergruppe (Carboxylschutzgruppe) abgespalten und mit dem aromatischen Alkohol oder Thiol umgesetzt.

Die oben beschriebene Herstellung der erfindungsgemäßen Substrate ist nicht erschöpfend und dem Fachmann stehen eine Reihe weiterer an sich bekannter Methoden zur Verfügung die es ihm ermöglichen, jede der erfindungsgemäßen Verbindungen ohne weiteres herzustellen. Aus den nach den oben erläuterten Verfahren erhaltenen racemischen Produkten können gewünschtenfalls die reinen optischen Isomeren nach bekannten Trennverfahren gewonnen werden. Ebenso lassen sich die Isomeren aber auch durch stereospezifisch ablaufende Synthese nach ebenfalls an sich bekannten Verfahren gewinnen.

Das erfindungsgemäße Verfahren zur optischen Bestimmung der Lipase ist dadurch gekennzeichnet, daß man ein erfindungsgemäßes Lipasesubstrat der Einwirkung der lipasehaltigen Probe unterwirft und die Menge der freigesetzten aromatischen Hydroxy- oder Thiolverbindung direkt oder nach Kopplung mit einem geeigneten Chromogen die daraus gebildete Farbe optisch bestimmt.

Ein besonderes Merkmal dieses Verfahrens besteht darin, daß es keine Hilfsenzyme oder Esterasehemmstoffe erfordert, wie sie bei den bekannten Methoden vielfach benötigt werden. Derartige Zusätze sind nicht nur teuer, sondern vielfach auch wenig stabil. Ein besonderer Vorzug der Erfindung liegt daher darin, daß sie auch ein einfaches und gut haltbares Reagens zur optischen Bestimmung der Lipase möglich macht, welches neben einem erfindungsgemäßen Lipasesubstrat und Puffersubstanz auch ein oberflächenaktives Mittel, wie insbesondere Gallensäuresalz, Colipase, einen chromogenen Kuppler oder/und ein Salz wie Natriumchlorid enthält. Außerdem kann das Reagens zweckmäßig auch Harnstoff, Konservierungsmittel oder/und Aktivator enthalten.

In einer bevorzugten Zusammensetzung enthält dieses Reagenz
0,05 bis 10 mg/ml Substrat,
2 bis 50 mg/ml Desoxycholat,
0,001 bis 0,01 mg/ml Colipase,
1 bis 100 mg/ml Harnstoff,
0,1 bis 10 mg/ml NaCl,
1 bis 50 mg/ml Puffersubstanz,
jeweils bezogen auf gebrauchsfertige Lösung im Test.

Als Gallensäure kommen die bekannten oberflächenaktiven Gallensäuren, wie Cholsäure, Taurocholsäure, Desoxycholsäure, Taurodesoxycholsäure, Glycodesoxycholsäure bzw. deren Alkalisalze, insbesondere das Natriumsalz, in Betracht. Die bevorzugte Menge beträgt 2 bis 50 mg/ml.

Ein weiterer wesentlicher Bestandteil des erfindungsgemäßen Reagenz ist Colipase. Besonders geeignet ist eine von Verunreinigungen freie Colipase. Die bevorzugte Menge beträgt 0,001 bis 0,01 mg/ml.

Das erfindungsgemäße Reagenz kann ferner Harnstoff, bevorzugt 1 bis 100 mg/ml, enthalten.

Als Puffersubstanz eignen sich alle bekannten Puffer, welche in der Lage sind, im Rahmen des erfindungsgemäßen Reagenz einen pH-Wert zwischen 6,0 und 10,5 einzustellen. Der bevorzugte pH-Wertbereich liegt zwischen 7,0 und 9,5. Beispiele für geeignete Puffer sind Diethanolaminpuffer, Triethanolaminpuffer, Tris-Puffer und Goodpuffer, wie Hepes-Puffer (gut geeignet zum Zusatz vor der Lyophilisation), Taps-Puffer, CHES-Puffer (2-(Cyclohexylamino)-ethansulfonsäure) und Bicine. Besonders bevorzugt wird Tris-Puffer. Die bevorzugte Puffersubstanzmenge liegt zwischen 1 und 50 mg/ml.

Als Salze sind Alkali-, Erdalkali- und Ammoniumsalze geeignet und werden bevorzugt in Konzentrationen von 0,1 bis 10 mg/ml eingesetzt.

Als Konservierungsmittel werden im Rahmen der Erfindung solche eingesetzt, die die enzymatische Aktivität der zu bestimmenden Lipase nicht beeinträchtigen. Besonders geeignet sind Alkaliazide, insbesondere Natriumazid. Auch andere Konservierungsmittel, wie z. B. Thiozid und andere schwefelhaltige Konservierungsmittel sind jedoch ebenfalls geeignet. Die bevorzugte Menge an Konservierungsmittel beträgt 0,001 bis 2 mg/ml.

Als Aktivatoren sind Erdalkaliionen, bevorzugt Calciumionen geeignet. Da diese mit Desoxycholsäure unlösliche Verbindungen eingehen, wird bei Gegenwart von Calcium als Gallensäure Taurodesoxycholsäure bevorzugt, da diese höhere Calciumkonzentrationen im Bereich von 1 bis 5 mMol zuläßt.

Wird das erfindungsgemäße Reagenz in trockener oder konzentrierter, zur Verdünnung auf die endgültige Zusammensetzung bestimmter Form eingesetzt, so enthält es die genannten Substanzen in entsprechenden Mengenverhältnissen, sowie vorzugsweise Schutzkolloid.

Als Schutzkolloid kommen die dem Chemiker hierfür bekannten Substanzen in Betracht, wie Polyhydroxyverbindungen, Serumalbumin, Polyvinylpyrrolidon, feste Polyethylenoxide und dergleichen. Bevorzugt werden Polyhydroxyverbindungen, insbesondere monomere oder polymere Pentose oder Hexose mit 1 bis 10 Pentose- bzw. Hexose-Einheiten im Molekül oder/und bei Zimmertemperatur fester Polyethylenglykol. Bevorzugte Beispiele von geeigneten Polyhydroxyverbindungen sind Mannit und ähnliche Zuckeralkohole, Oligosaccharid der Glucose, Mannose, Maltoheptaose, Polyethylenglykol mit einem mittleren Molekulargewicht zwischen 3500 und 7000 u. ä. Andere verwendbare Schutzkolloide sind z. B. Aminosäuren, wie

Alanin, Pflanzengummis, wie Gummi arabicum usw. Die bevorzugte Menge an Schutzkolloid bzw. einer Mischung von Schutzkolloiden beträgt 20 bis 90 Gew.-%. Als besonders geeignet erwies sich eine Mischung von Zuckeralkohol und Polyalkylenglykol.

Das erfindungsgemäße Reagenz kann auch auf einem geeigneten Trägermaterial imprägniert vorliegen. In betracht kommt sowohl ein saugfähiges Trägermaterial, als auch ein quellfähiges, lösliches filmbildendes Trägermaterial. In dieser Form ermöglicht das erfindungsgemäße Reagenz die Herstellung von Teststreifen, welche direkt visuell oder mittels geeigneter Meßgeräte ausgewertet werden können.

Der erfindungsgemäße Farbtest zur Bestimmung der Lipase liefert sehr genaue Ergebnisse bei hoher Empfindlichkeit. Er ist sehr einfach in der Handhabung und eignet sich sogar für Teststreifen. Da er nur eine sehr geringe oder gar keine lag-Phase aufweist, kann er auf die verschiedenen Analysenautomatensysteme ohne weiteres adaptiert werden.

Die Bestimmung selbst kann sowohl als Endpunktbestimmung als auch kinetisch durchgeführt werden. Gegenüber vielen bekannten Verfahren liegt in der kinetischen Durchführbarkeit der Vorteil, daß weder ein Abstoppen, noch ein Ausschütteln des gebildeten Reaktionsproduktes durchgeführt werden muß.

Die folgenden Beispiele erläutern die Erfindung weiter.

**Beispiel 1**

a) 1,2-0-Dihexyl-rac-glycero-3-glutarsäure-monoester

Zu einer Lösung von 3,3 g (11,5 mM) 1,2-0-Dihexylglycerin in 30 ml Chloroform werden nacheinander 2,5 ml Pyridin, eine Spatelspitze 4-Dimethylaminopyridin und 2,6 g (23 mM) Glutarsäureanhydrid zugefügt. Das Gemisch wird 10 Stunden unter Rückfluß erhitzt und nach dem Abkühlen mit 200 ml Chloroform verdünnt. Die Chloroformphase wird mit 1N Salzsäure geschüttelt und getrocknet. Nach Abfiltrieren des Trockenmittels wird das Lösungsmittel abgezogen und der Rückstand über eine Kieselgelsäule (Eluens: Essigester/Petrolether 1:1) gereinigt.

DC: $R_f$: 0,45 (Essigester/Petrolether 1:2 + 1 % Eisessig)

1,2-0-Dihexyl-rac-glycero-3-glutarsäure-resorufinester:

b) 1,4 g (3,7 mM) 1a) werden in 20 ml Chloroform gelöst und unter Eiskühlung mit 2 ml (23,3 mM) Oxalylchlorid zugetropft. Das Eisbad wird entfernt und die Lösung 12 Stunden bei Raumtemperatur gerührt. Anschließend wird das Lösungsmittel abgezogen, der Rückstand in Toluol aufgenommen und wieder eingedampft. Das so erhaltene Öl wird ohne weitere Reinigung eingesetzt.

c) 0,8 g (3,7 mM) Resorufin werden in 40 ml Dimethylformamid unter Zusatz von 1,1 ml Pyridin und einer Spatelspitze 4-Dimethylaminopyridin aufgeschlämmt. Hierzu wird eine Lösung von 1 b) in 20 ml Dimethylformamid getropft. Nach ein- bis zweitägigem Rühren bei Raumtemperatur filtriert und das Lösungsmittel abgezogen. Der Rückstand wird im Essigester aufgenommen, unlösliche Bestandteile abfiltriert und das Filtrat mit 1 N Salzsäure, dann mit Wasser geschüttelt. Nach dem Trocknen der organischen Phase und Abdestillieren des Lösungsmittels erhält man einen öligen Rückstand, der über Säulenchromatographie an Kieselgel gereinigt wird (Eluens: Essigester/ Petrolether 1:1).

DC: $R_f$: 0,70 (Essigester/Hexan 1:1).

**Beispiel 2**

a) 1,2-0-Dioctyl-rac-glycero-3-glutarsäure-monoester

Herstellung analog Beispiel 1a) aus 13 g (41 mM) 1,2-0-Dioctyl-glycerin, 150 ml Chloroform, 10 ml Pyridin und 6,8 g (59,5 mM) Glutarsäureanhydrid.

Ausbeute: 6,5 g (37%)

DC: $R_f$: 0,31 (Essigester/Petrolether 1:1).

1,2-0-Dioctyl-rac-glycero-3-glutarsäure-resorufinester:

b) Herstellung analog Beispiel 1 b) aus 2 g (4,5 mM) 2 a)

c) 0,96 g (4,5 mM) Resorufin werden in 50 ml Chloroform unter Zusatz von 0,75 ml (5 mM) 1,8-Diazabicyclo-(5,4,0)-undec-7-en und 0,1 g 4-Dimethylaminopyridin gelöst. Hierzu wird eine Lösung von 2 b) in 20 ml Chloroform getropft. Nach ein- bis zweitägigem Rühren bei Raumtemperatur wird filtriert und das Lösungsmittel abgezogen. Aufarbeitung analog Beispiel 1 c).

DC: $R_f$: 0,66 (Essigester/Hexan 1:1).

**Beispiel 3**

a) 1,2-0-Dioctyl-rac-glycero-3-pimelinsäure-monoester

Herstellung analog Beispiel 1a) aus 3,2 g (10 mM) 1,2-0-Dioctyl-glycerin und 2,1 g (15 mM) Pimelinsäureanhydrid,
DC: $R_f$: 0,61 (Essigester/Petrolether 1:1)
IR ($cm^{-1}$): (Film): 1740, 1710
1,2-0-Dioctyl-rac-glycero-3-pimelinsäure-resorufinester:
b) Herstellung analog Beispiel 1b) aus 2,2 g (4,7 mM) 3 a)
c) Herstellung analog Beispiel 2c) aus 1 g (4,7 mM) Resorufin, 0,7 ml 1,8-Diazabicyclo-(5,4,0)-undec-7-en und 3b).
DC: $R_f$ = 0,78 (Essigester/Hexan 1:2)

**Beispiel 4**

a) 1,2-0-Dioctyl-rac-glycero-3-azelainsäure-monoester
Herstellung analog Beispiel 1a) aus 6,3 g (20 mM) 1,2-0-Dioctyl-glycerin, 80 ml Chloroform, 5 ml Pyridin und 5,2 g (30 mM) Azelainsäureanhydrid. 1,2-0-Dioctyl-rac-glycero-3-azelainsäure-resorufinester:
b) Herstellung analog Beispiel 1 b) aus 3,2 g (6,5 mM) 4a) und 3 ml Oxalylchlorid.
c) Herstellung analog Beispiel 2 c) aus 1,4 g (6,5 mM) Resorufin, 65 ml Chloroform 1,1 ml 1,8-Diazabicyclo-(5,4,0)-undec-7-en und 4b).
DC: $R_f$ = 0,86 (Essigester/Hexan 1:2)
IR ($cm^{-1}$): (Film) 1762, 1736

**Beispiel 5**

a) 1,2-0-Didecyl-rac-glycero-3-glutarsäure-monoester
Herstellung analog Beispiel 1 a) aus 3,7 g (10 mM) 1,2-0-Didecyl-glycerin, 40 ml Chloroform, 2,5 ml Pyridin und 1,8 g (15,8 mM) Glutarsäureanhydrid.
DC: $R_f$: 0,77 (Essigester/Hexan 1:2)
1,2-0-Didecyl-rac-glycero-3-glutarsäure-resorufinester:
b) Herstellung analog Beispiel 1 b) aus 2,5 g (5 mM) 5 a) 50 ml Chloroform und 2,2 ml Oxalylchlorid.
c) Herstellung analog Beispiel 2 c) aus 1,1 g (5 mM) Resorufin, 1 ml 1,8-Diazabicyclo-(5,4,0)-undec-7-en und 5 b).
DC: $R_f$ = 0,70 (Essigester/Hexan 1:1)

**Beispiel 6**

a) 1,2-0-Diundecyl-rac-glycero-3-glutarsäure-monoester
Herstellung analog Beispiel 1a) aus 2 g (5 mM) 1,2-0-Diundecyl-glycerin, 25 ml Chloroform, 1,4 ml Pyridin und 0,9 g (7,5 mM) Glutarsäureanhydrid.
DC: $R_f$: 0,55 (Essigester/Petrolether 1:2)
IR ($cm^{-1}$): (Film) 1740, 1710
1,2-0-Diundecyl-rac-glycero-3-glutarsäure-resorufinester:
b) Herstellung analog Beispiel 1 b) aus 2 g (3,9 mM) 6 a), 5 ml Chloroform und 1,8 ml Oxalylchlorid.
c) Herstellung analog Beispiel 2 c) aus 0,83 g (3,9 mM) Resorufin, 0,61 ml 1,8-Diazabicyclo-(5,4,0)-undec-7-en und 6 b).
DC: $R_f$ = 0,47 (RP 18, Ethanol/Aceton 2:1)

**Beispiel 7**

a) 1,2-0-Dilauryl-rac-glycero-3-glutarsäure-monoester
Herstellung analog Beispiel 1 a) aus 10,7 g (25 mM) 1,2-0-Dilauryl-glycerin, 70 ml Chloroform, 5,5 ml Pyridin und 3,3 g (29 mM) Glutarsäureanhydrid.
DC: $R_f$: 0,33 (Essigester/Petrolether 1:1).
IR ($cm^{-1}$): (Film): 1741, 1708
1,2-0-Dilauryl-rac-glycero-3-glutarsäure-resorufinester
b) Herstellung analog Beispiel 1 b) aus 5,5 g (10 mM) 7 a), 50 ml Chloroform und 4,3 ml Oxalylchlorid.
c) Herstellung analog Beispiel 2 c) aus 2,2 g (10 mM) Resorufin, 100 ml Chloroform und 1,5 ml 1,8-Diazabicyclo-(5,4,0)-undec-7-enund 7 b).
DC: $R_f$ = 0,78 (Essigester/Petrolether 1:1)

IR ($cm^{-1}$): (Film) 1765, 1720

**Beispiel 8**

a) 1,2-0-Dilauryl-rac-glycero-3-pimelinsäure-monoester
Herstellung analog Beispiel 1a) aus 8,6 g (20 mM) 1,2-0-Dilauryl-glycerin, 50 ml Chloroform, 15 ml Pyridin und 4,3 g (30 mM) Pimelinsäureanhydrid.
DC: $R_f$: 0,5 (Essigester/Petrolether 1:2).
IR ($cm^{-1}$): (Film): 1740, 1710
1,2-0-Dilauryl-rac-glycero-3-pimelinsäure-resorufinester:
b) Herstellung analog Beispiel 1 b) aus 1,66 g (3 mM) 8 a) und 1,3 ml Oxalylchlorid.
c) Herstellung analog Beispiel 2 c) aus 0,65 g (3 mM) Resorufin, 30 ml Chloroform 0,5 ml 1,8-Diazabicyclo-(5,4,0)-undec-7-en und 8 b).
DC: $R_f$ = 0,75 (Essigester/Hexan 1:1)
IR ($cm^{-1}$): (Film): 1768, 1739

**Beispiel 9**

a) 1,2-0-Ditetradecyl-rac-glycero-3-glutarsäure-monoester
Herstellung analog Beispiel 1 a) aus 14,6 g (30 mM) 1,2-0-Ditetradecyl-glycerin, 150 ml Chloroform, 8,2 ml Pyridin und 5,1 g (45 mM) Glutarsäureanhydrid.
DC: $R_f$: 0,42 (Essigester/Petrolether 1:2)
IR ($cm^{-1}$): (KBr) 1740, 1710
1,2-0-Ditetradecyl-rac-glycero-3-glutarsäureresorufinester:
b) Herstellung analog Beispiel 1 b) aus 3 g (5 mM) 9 a) und 2,2 ml Oxalylchlorid.
c) Herstellung analog Beispiel 2c) aus 1,06 g (5 mM) Resorufin, 50 ml Chloroform, 0,75 ml 1,8-Diazabicyclo-(5,4,0)-undec-7-en und 9 b).
DC: $R_f$ = 0,34 (RP18, Acetonitril/Dichlormethan 1:1)
IR ($cm^{-1}$): (KBr): 1763, 1735

**Beispiel 10**

a) 1,2-0-Ditetradecyl-rac-glycero-3-pimelinsäuremonoester
Herstellung analog Beispiel 9 a) aus 6,4 g (45 mM) Pimelinsäureanhydrid
DC: $R_f$: 0,45 (Essigester/Petrolether 1:2)
IR ($cm^{-1}$): (Film) 1740, 1708
1,2-0-Ditetradecyl-rac-glycero-3-pimelinsäureresorufinester
b) Herstellung analog Beispiel 1 b) aus 3,1 g (5 mM) 10 a) und 2,2 ml Oxalylchlorid.
c) Herstellung analog Beispiel 2 c) aus 1,06 g (5 mM) Resorufin, 50 ml Chloroform und 0,78 ml 1,8-Diazabicyclo-(5,4,0)-undec-7-enund 10 b).
DC: $R_f$ = 0,71 (Essigester/Petrolether 1:2)
IR ($cm^{-1}$): (Film): 1755, 1734

**Beispiel 11**

a) 1,2-0-Dihexadecyl-sn-glycero-3-glutarsäure-monoester
Herstellung analog Beispiel 1 a) aus 2,7 g (5 mM) 1,2-0-Dihexadecyl-sn-glycerin, 50 ml Chloroform, 3 ml Pyridin und 1,5 g (13 mM) Glutarsäureanhydrid.
DC: $R_f$: 0,65 (Essigester/Petrolether 1:1)
IR ($cm^{-1}$): (KBr) 1740, 1710
1,2-0-Dihexadecyl-sn-glycero-3-glutarsäure-resorufinester:
b) Herstellung analog Beispiel 1 b) aus 2,2 g (3,3 mM) 11 a) und 1 ml Oxalylchlorid.
c) Herstellung analog Beispiel 1 c) aus 0,71 g (3,3 mM) Resorufin, 20 ml Dimethylformamid 0,5 ml Pyridin und 11 b).
DC: $R_f$ = 0,72 (Essigester/Petrolether 1:2).

**Beispiel 12**

a) 1,2-0-Dibenzyl-rac-glycero-3-glutarsäure-monoester
Herstellung analog Beispiel 1 a) aus 3 g (11 mM) 1,2-0-Dibenzyl-glycerin, 30 ml (Chloroform, 2,5 ml Pyridin und 1,8 g (16 mM) Glutarsäureanhydrid.
DC: $R_f$: 0,39 (Essigester/Petrolether 1:1 + 1% Eisessig)
1,2-0-Dibenzyl-rac-glycero-3-glutarsäure-resorufinester:
b) Herstellung analog Beispiel 1 b) aus 2,9 g (7,5 mM) 12 a), 30 ml Chloroform und 3,3 ml Oxalylchlorid.
c) Herstellung analog Beispiel 2 c) aus 1,6 g (7,5 mM) Resorufin, 75 ml Chloroform, 1,2 ml 1,8-Diazabicyclo-(5,4,0)-undec-7-en und 12 b).
DC: $R_f$ = 0,48 (Essigester/Hexan 1:1)

**Beispiel 13**

a) 1-0-Octadecyl-2-0-benzyl-sn-glycero-3-glutarsäuremonoester
Herstellung analog Beispiel 1 a) aus 2,2 g (5 mMol) 1-0-Octadecyl-2-0-benzyl-sn-glycerin, 50 ml Chloroform, 3 ml Pyridin und 1,5 g (13 mM) Glutarsäureanhydrid.
IR ($cm^{-1}$): (Film) 1730, 1700
1-0-Octadecyl-2-0-benzyl-sn-glycero-3-glutarsäureresorufinester:
b) Herstellung analog Beispiel 1 b) aus 1,7 g (3,1 mM) 13 a) und 1,3 ml Oxalylchlorid.
c) Herstellung analog Beispiel 1 c) aus 0,8 g (37 mM) Resorufin, 20 ml Dimethylformamid, 0,7 ml Pyridin und 13 b).
DC: $R_f$ = 0,68 (Essigester/Petrolether 1:1)
IR ($cm^{-1}$). (KBr): 1768, 1739

**Beispiel 14**

a) 1,2-Dioctanoyl-sn-glycero-3-glutarsäure-monoester
Herstellung analog Beispiel 1 a) aus 6,8 g (20 mM) 1,2-Dioctanoyl-sn-glycerin, 100 ml Chloroform, 12,5 ml Pyridin und 5,8 g (50 mM) Glutarsäureanhydrid.
DC: $R_f$: 0,49 (Essigester/Petrolether 1:1).
1,2-Dioctanoyl-sn-glycero-3-glutarsäure-resorufinester
b) Herstellung analog Beispiel 1 b) aus 2,2 g (5 mM) 14 a) und 2,2 ml Oxalylchlorid
c) Herstellung analog Beispiel 1 c) aus 1,05 g (5mM) Resorufin, 30 ml Dimethylformamid, 0,75 ml Pyridin und 14 b).
DC: $R_f$ = 0,86 (RP18, Acetonitril/Dichlormethan 1:2)

**Beispiel 15**

a) 1.2.-Dioleyl-rac-glycero-3-glutarsäure-monoester
Herstellung analog Beispiel 1 a) aus 3,1 g (5 mMol) Diolein, 40 ml Chloroform, 3 ml Pyridin und 1,5 g (13 mM) Glutarsäureanhydrid.
DC: $R_f$: 0,32 (Essigester/Petrolether 1:1)
IR ($cm^{-1}$): (Film): 1740, 1706
Diolein läßt sich aus technischen Diolein durch Säulenchromatographie an Kieselgel unter Verwendung von Essigester/Petrolether 1:3 als Laufmittel in reiner Form herstellen.
1.2.-Dioleyl-rac-glycero-3-glutarsäure-resorufinester:
b) Herstellung analog Beispiel 1b) aus 3,5 g (4,8 mM) 15 a) und 1,3 ml Oxalylchlorid.
c) Herstellung analog Beispiel 1 c) aus 0,9 g (4,2 mM) Resorufin, 20 ml Dimethylformamid, 1 ml Pyridin und 15 b).
DC: $R_f$ = 0,78 (Essigester/Petrolether 1:1)

**Beispiel 16**

1,2-0-Ditetradecyl-rac-glycero-pimelinsäure-naphthylester

Die Herstellung erfolgt analog Beispiel 10 b) und c) aus 0,72 g (5 mM) 1-Naphthol. Aufreinigung über Flash-Chromatographie an Kieselgel mit dem Laufmittel Essigester/Hexan 1:3.
DC: $R_f$ = 0,89 (Essigester/Petrolether 1:5)

**Beispiel 17**

1.2.-Dioleyl-rac-glycero-3-glutarsäure-resorufinester

10,3 g (14,2 mM) 1.2-Dioleyl-3-glyceroglutarsäuremonoester nach Beispiel 15 a) , 3,2 g (15 mM) Resorufin, 6,2 g (30 mM) Dicyclohexylcarbodiimid und eine Spatelspitze 4-Dimethylaminopyridin rührt man in 75 ml Dimethylformamid 2 bis 3 Tage bei Raumtemperatur. Dann wird mit Essigester verdünnt und der Niederschlag abfiltriert. Die Essigesterphase wird mit 1 N Salzsäure ausgeschüttelt und über Natriumsulfat getrocknet. Nach Abdestillieren des Lösungsmittels läßt ein öliger Rückstand, der über Kieselgel- Säulenchromatographie (Laufmittel: Essigester/Petrolether 1:1) gereinigt wird. Ebenso wurde das entsprechende Chlorphenolrot-Derivat hergestellt.
DC; $R_f$ = 0,69 (RP 18; Isopropanol/Methanol 1:2).

**Beispiel 18**

Herstellung von 1,2-0-Dioctyl-3-pimelinsäure-monoester

1. Stufe: Lit: J. H. Short, U. Biermacher Chim. Ther. 1966, 456 Synthese von Pimelinsäuremonobenzylester
2. Stufe: Analog Beispiel 1 b) aus 2,5 g (10 mM) Pimelinsäuremonobenzylester. Das erhaltene Öl wird zu einer Lösung von 3,2 g (10 mM) 1,2-0-Dioctylglycerin, 7 ml Pyridin getropft. Aufarbeitung analog Beispiel 1 c).
3. Stufe: Obiges Produkt wurde in 20 ml Tetrahydrofuran gelöst und nach Zusatz von 0,4 g Palladium/Aktivkohle hydriert. Das Rohprodukt wird über eine Kieselgel-Säule gereinigt.
Laufmittel: Essigester/Petrolether 1:1.
DC: $R_f$ = 0,61 (Essigester/Petrolether 1:1)
IR ($cm^{-1}$): (Film): 1740, 1710.

**Beispiel 19**

Synthese des 1.2.-Dioleyl-glycero-3-glutarsäuremonoesters

a) 1,2-0-Isopropyliden-glycero-3-glutarsäure-trichlorethylester

Stufe 1: 5,55 g (42 mM) Isopropylidenglycerol und 12 g (45,3 mM) 2,2,2-Trichlorethyl-hydrogenglutarat werden in Ethylenglycoldimethylether gelöst und mit 10,5 g (51mM) Dicyclohexylcarbodiimid versetzt. Nach zweitägigem Rühren wird filtriert und destilliert.
Sdp: 170 °C (0,1 Torr) farbl. Öl
DC: $R_f$ = 0,82
(Aceton/Chloroform 1:8)

Stufe 2: Das erhaltene Öl wird in 11 ml Ether gelöst, mit 3 ml Methanol und 3 ml 3N Salzsäure versetzt und 12 Stunden bei Raumtemperatur gerührt. Die organische Phase wird mit gesättigter Natriumhydrogencarbonatlösung, dann mit gesättigter Natriumchloridlösung geschüttelt und getrocknet. Nach Abziehen des Lösungsmittels bleibt ein öliger Rückstand.
DC: $R_f$ = 0,23 (Essigester/Petrolether 1:1)

b) 1,2-Dioleyl-glycero-3-glutarsäuretrichlorethylester

Stufe 3: 6,1 g (18 mM) des oben erhaltenen Produkts und 7,84 g (38 mM) Ölsäure werden in 100 ml Ethylenglycoldimethylether gelöst und eine Lösung von 10,5 g (37 mM) Dicyclohexylcarbodiimid in 50 ml Ethylenglycoldimethylether zugetropft. Nach 12stündigem Rühren bei Raumtemperatur wird filtriert und nacheinander mit 3 N Salzsäure, mit Natriumhydrogencarbonatlösung, dann mit Wasser ausgeschüttelt. Nach Trocknen und Einengen der organischen Phase wird der Rückstand an Kieselgel chromatographiert.

Stufe 4 1.2-Dioleyl-glycero-3-glutarsäure-monoester
Abspaltung der Trichlorethyl-Schutzgruppe nach Literaturvorschrift: Juste, Synthesis 1976, 457.

**Beispiel 20**

a) 1,2-0-Didecyl-rac-glycero-3-pimelinsäure-monoester
Herstellung analog Beispiel 1a) aus 5,6 g (15 mM) 1,2-0-Didecyl-glycerin, 100 ml Chloroform, 3,2 ml Pyridin und 4,0 g (28 mM) Pimelinsäureanhydrid.
DC: $R_f$ = 0,36 (Essigester/Hexa 1:1)
IR ($cm^{-1}$): (Film) 1735, 1710
1,2-0-Didecyl-rac-glycero-3-pimelinsäure-resorufinester
b) Herstellung analog Beispiel 1b) aus 3 g (5,8 mM) 20a, 50 ml Chloroform und 2,2 ml Oxalylchlorid c) Herstellung analog Beispiel 2c) aus 1,1 g (5 mM) Resorufin, 1 ml (6,4 mM) 1,8-Diazabicyclo-(5,4,0)-undec-7-en und 20b.
DC: $R_f$ = 0,71 (Essigester/Hexan 1:1)
IR ($cm^{-1}$): (Film) 1755, 1720

**Beispiel 21**

a) 1,2-0-Dilauryl-rac-glycero-3-azelainsäure-monoester
Herstellung analog Beispiel 1a) aus 6,7 g (15,6 mM) 1,2-0-Dilaurylglycerin, 100 ml Chloroform, 3,2 ml Pyridin und 4,0 g (23 mM) Azelainsäureanhydrid.
DC: $R_f$ = 0,22 (Essigester/Hexan 1:1)
IR ($cm^{-1}$): (Film): 1738, 1710
1,2-0-Dilauryl-rac-glycero-3-azelainsäure-resorufinester
b) Herstellung analog Beispiel 1b) aus 1,5 g (2,5 mM) 21a, 30 ml Chloroform und 1,5 ml Oxalylchlorid
c) Herstellung analog Beispiel 2c) aus 0,55 g (2,5 mM) Resorufin, 25 ml Chloroform, 0,5 ml (3,2 mM) 1,8-Diazabicyclo-(5,4,0)-undec-7-en und 21b
DC: $R_f$ = 0,78 (Essigester/Hexan 1:1)
IR ($cm^{-1}$): (Film): 1762, 1740

**Beispiel 22**

In 60 ml destilliertem Wasser werden unter Rühren 1,2 g Natrium-Desoxycholat und 0,15 mg Colipase (vom Schwein) gelöst. Unter heftigem Rühren wird hierin eine Lösung von 70 mg Lipasesubstrat 1,2-0-Dioctyl-rac-glycero-3-azelainsäure-resorufinester (Beispiel 4) in 1,7 ml n-Propanol unter Druck in einem möglichst dünnen Strahl eingespritzt. Eine Lösung, die in 200 ml destilliertem Wasser 1,5 g Harnstoff, 1 g Natrium-Desoxy-Cholat, 200 mg Natriumchlorid, 800 mg TRIS und 107 mg TRIS.HCl gelöst enthält, wird mit der oben hergestellten Emulsion gut gemischt.

2,5 ml der so hergestellten Lösung werden mit 100 µl Probe (Serum) versetzt. Die Reaktion wird bei 578 nm Hg photometrisch verfolgt.

Bei Auswertung über einen Standard bekannter Lipaseaktivität wird die Lipaseaktivität der Probe wie folgt berechnet:

$$\text{Aktivität}_{(\text{Probe})} \quad \frac{\text{Aktivität}_{(\text{Standard})} \cdot \Delta E/\text{min (Probe)}}{\Delta\, E/\text{min (Standard)}}$$

Eine Berechnung der Lipaseaktivität der Probe ist auch nach folgender Formel möglich:

$$\text{Aktivität (Probe) } [U/l] = 1000 \cdot \frac{V_{ges}}{\varepsilon \cdot V_{Probe} \cdot d} \cdot \Delta E/\text{min}$$

$V_{ges}$: Gesamtvolumen des Testansatzes [$cm^3$]
$V_{Probe}$ : Volumen der Probe [$cm^3$]
$\epsilon$ : Extinktionskoeffizient des Chromogens bei 578 nm
d : Schichtdicke der Küvette [cm]

ΔE/min: Extinktionsänderung pro Minute bei 578 nm

Bei den genannten Reaktionsbedingungen beträgt der Extinktionskoeffizient $\epsilon$ = 60,65 cm . $\mu$mol$^{-1}$.

**Beispiel 23**

Für verschiedene Lipasesubstrate wurden Leerwertänderung, Esteraseempfindlichkeit, Lipaseempfindlichkeit und Korrelation zu einem turbidimetrischen Trübungstest bestimmt.

Die Leerwertänderung wurde ermittelt mit einem Reagenz gemäß Beispiel 1 und verschiedenen Lipasesubstraten. Anstelle der Probe wurden 100 $\mu$l Wasser zugegeben und die Extinktionsänderung bei 578 nm photometrisch verfolgt (ΔmE/min).

Zur Ermittlung der Esteraseempfindlichkeit wurde anstelle der Probe 100 $\mu$l Carboxylesterase (EC 3.1.1.1, ca. 20 000 U/1 Boehringer Mannheim GmbH, Best.-Nr. 10 46 98) zugegeben und die Extinktionsänderung wie oben beschrieben verfolgt.

Zur Ermittlung der Lipaseempfindlichkeit wurde anstelle der Probe 100 $\mu$l Lipase (EC 3.1.1.3, ca. 100 U/l, Boehringer Mannheim GmbH, Best.-Nr. 41 45 90) zugegeben und die Extinktionsänderung wie oben beschrieben verfolgt.

Zur Ermittlung der Korrelation zu einem turbidimetrischen Trübungstest wurde mit steigenden Lipasemengen (0 - 1000 U/l) ein Trübungstest (Boehringer Mannheim GmbH, Best.-Nr. 26 23 58) durchgeführt mit einem Farbtest gemäß Beispiel 22 verglichen und der Korrelationskoeffizient ermittelt.

Die Ergebnisse zeigt Tabelle I:

Tabelle I

| Beispiel | Leerwert [ΔmE/min] | Esteraseempfindlichkeit (Aktivität 20 000 U/l) [ΔmE/min] | Lipaseempfindlichkeit (Aktivität 100 U/l) [ΔmE/min] | Korrelationskoeffizient |
|---|---|---|---|---|
| 1 | 13,2 | > 1000 | - | - |
| 2 | 7,0 | 144,0 | ~ 100 | nicht ermittelt |
| 3 | 3,9-4,1 | 112,5 | 12,28 | 0,9639 |
| 4 | 0,4-0,8 | 33,6 | 18,04 | 0,9421 |
| 5 | 2,4-3,0 | 30,2 | 22,18 | 0,9815 |
| 6 | 2,0-2,8 | 23,6 | 6,96 | 0,9179 |
| 7 | 2,4-3,0 | 34,8 | 10,9 | 0,9636 |
| 8 | 2,1-2,4 | 27,8 | 4,68 | 0,9286 |
| 11 | 0,1 | 1,8 | 0,97 | 0,9830 |
| 20 | 1,3-2,0 | 48,7 | 9,8 | 0,9888 |
| 21 | 9,8-11,9 | 9,5 | 13,2 | 0,9716 |

**Beispiel 24**

8,5 g Natriumdesoxycholat, 0,05 g Colipase, 20 g Mannit, 0,05 g Calciumchlorid, 0,82 g Natriumchlorid, 2,7 g TRIS und 0,4 g TRIS.HCl werden in 200 ml destilliertem Wasser gelöst. Unter Rühren wird eine Lösung, die 0,35 g 1,2-o-Ditetradecyl-rac-glycero-3-pimelinsäure-naphtholester in 7 ml Propanol gelöst

enthält, eingespritzt. Die so erhaltene Emulsion wird bei -40 ° C eingefroren und lyophilisiert.
70 mg des erhaltenen Lyophilisates werden in 2 ml destilliertem Wasser gelöst und mit 100 µl einer Echtrot TR-Lösung (Echtrot = 4-Chlor-3-methyl-benzoldiazonium Naphthalinl,5-disulfonat) (231 mg in 10 ml destilliertem Wasser) versetzt.
Nach Zugabe von 100 µl Probe (Serum) wird die Reaktion bei 405 nm photometrisch verfolgt.
Die Ermittlung der Lipasekonzentration erfolgt über eine Eichkurve analog zu Beispiel 22.

**Beispiel 25**

In eine Lösung von 3,04 g Taurodesoxycholat, 2,7 g Polywachs 4000, 7 mg Calciumchlorid, 0,2 mg Colipase (vom Schwein) in 120 ml dest. Wasser werden unter Rühren 150 mg Lipasesubstrat 1,2-0-Didecyl-rac-glycero-3-glutarsäure-resorufinester in 3,5 ml n-Propanol eingespritzt.

Zu der so erhaltenen Emulsion wurde die folgende Lösung zugegeben und gut gemischt:
10 g Taurodesoxycholat, 6,4 g Polywachs, 50 g Mannit, 14 g Harnstoff, 800 mg Natriumchlorid und 15 g Tris werden in 300 ml dest. Wasser gelöst. Mit einer Salzsäurelösung wird der pH-Wert auf pH = 7,5 eingestellt. Danach wird die Lösung auf 400 ml mit dest. Wasser aufgefüllt.

2,5 ml des so hergestellten Reaktionsgemisches werden mit 100 µl Probe (Serum) versetzt. Die Reaktion wird bei 578 nm photometrisch verfolgt.

Die Auswertung wird wie im Beispiel 22 vorgenommen.

**Beispiel 26**

In eine Lösung von 3,04 g Taurodesoxycholat, 2,7 g Polywachs 4000, 7 mg Calciumchlorid, 0,2 mg Colipase (vom Schwein) in 120 ml dest. Wasser werden unter Rühren 150 mg Lipasesubstrat 1,2-0-Didecyl-rac-glycero-3-glutarsäure-resofurinester in 3,5 ml n-Propanol eingespritzt.

Zu der so erhaltenen Emulsion wurde die folgende Lösung zugegeben und gut gemischt:
10 g Taurodesoxycholat, 6,4 g Polywachs, 50 g Mannit, 14 g Harnstoff, 800 mg Natriumchlorid, 28 g CHES werden in 300 ml dest. Wasser gelöst. Mit einer Salzsäurelösung wird der pH-Wert auf pH = 8,5 eingestellt. Danach wird die Lösung auf 400 ml mit dest. Wasser aufgefüllt.

2,5 ml des so hergestellten Reaktionsgemisches werden mit 100 µl Probe (Serum) versetzt. Die Reaktion wird bei 578 nm photometrisch verfolgt.

Die Auswertung wird wie im Beispiel 22 angegeben, vorgenommen.

**Beispiel 27**

In eine Lösung aus 4,0 g Na-Taurodesoxycholat, 0,06 g Calciumchlorid, 0,2 mg Colipase (vom Schwein), 5,0 g Mannit und 2,0 g Polywachs 4000 in 100 ml dest. Wasser wird unter Rühren 150 ml 1,2-o-Ditetradecyl-rac-glycero-3-pimelinsäure-naphtholester, der in 4 ml n-Propanol gelöst ist, eingespritzt. Unter guter Kühlung wird die so erhaltene Emulsion einige Minuten mit Ultraschall behandelt.

Eine zweite Lösung enthält in 100 ml 2,4 g Na-Taurodesoxycholat, 2,0 g TRIS, 12,0 g Mannit, 3,5 g Harnstoff und 0,5 g Natriumchlorid. Mit Salzsäure wird der pH-Wert dieser Lösung auf pH = 8,3 eingestellt.

Eine dritte Lösung enthält 1 g Echtrot TR (Echtrot TR = 4-Chlor-3-methyl-benzol-diazonium-naphthalin-1,5-disulfonat) in 40 ml dest. Wasser gelöst.

5 Teile der Lösung 1 und 6 Teile der Lösung 2 werden mit einem Teil der Lösung 3 gemischt. Mit der so erhaltenen Lösung wird ein zur Teststreifenherstellung geeignetes saugfähiges Papier, z. B. die Sorte VS 532 der Firma Schleicher und Schüll getränkt und in einem Umlufttrockenschrank bei einer Temperatur von 30 bis 65 ° C schonend getrocknet.

Bis zur weiteren Verwendung empfiehlt sich eine Aufbewahrung in Gegenwart eines feuchtigkeitsentziehenden Mittels.

Zur Prüfung auf Lipase der Probe (Serum) wird eine kleine Menge (einige Tropfen) des Probenmaterials auf den Streifen gegeben. Aus dem zeitlichen Verlauf der Gelbfärbung kann auf die Lipaseaktivität der zu untersuchenden Probe geschlossen werden.

**Beispiel 28**

a) 1,2-0-Dilauryl-rac-glycero-3-tetradecandisäuremonoester:
Herstellung analog Beispiel 1a) aus 8.6 g (20 mM) 1,2-0-Dilaurylglycerin, 100 ml Chloroform, 4 ml Pyridin, 0,4 g Dimethylaminopyridin, 9,6 g (40 mM) Tetradecandisäureanhydrid.

DC: $R_f$ = 0.45 (Essigester/Petrolether 1:5)
1,2-0-Dilauryl-rac-glycero-3-tetradecandisäure (6-methylresorufin-)ester:
b) Herstellung analog Beispiel 1b) aus 3.35 g (5mM) 28a) und 2.2 ml Oxalylchlorid
c) Herstellung analog Beispiel 2c) aus 1,2g (5mM) 4-Methylresorufin, 20 ml Chloroform, 0.75 ml 1,8-Diazabicyclo-(5,4,0)-undec-7-en, 0.1 g Dimethylaminopyridin und 28b).
DC: $R_f$ = 0.63 (Essigester/Hexan 1:4)
d) Wie in Beispiel 22 angegeben wird eine Emulsion hergestellt. Jedoch wird anstelle des dort genannten Lipasesubstrats eine Lösung von 70 mg Lipasesubstrat 1,2-0-Dilauryl-rac-glycero-3-tetradecandisäure-(6-methylresorufin)-ester in 1,7 ml n-Propanol gelöst eingesetzt.

Dabei wurden die folgenden testspezifischen Kenngrößen erhalten (vgl. Beispiel 23 und Tabelle I):

| | |
|---|---|
| Leerwert: | 0,2 mE/min |
| Esteraseaktivität: | 0,7 mE/min |
| Lipaseempfindlichkeit: | 23,6 mE/min pro 100U/l |
| Korrelationskoeffizient: | 0,09509 |

**Beispiel 29**

1,2-0-Dilauryl-rac-glycero-3-glutarsäure-(6-methyl-resorufin-)ester:

a) Herstellung analog den Beispielen 7a-c mit 2.3g (10 mM) 4-Methylresorufin.
DC: $R_f$ = 0,68 (Essigester/Hexan 1:2)
b) In 60 ml dest. Wasser werden unter Rühren 0,9 g Natrium-Taurodesoxycholat und 0,3 g Colipase (vom Schwein) gelöst. Unter gutem Rühren wird hierin eine Lösung von 1,2-O-Dilauryl-rac-glycero-3-glutarsäure-(6-methyl-resorufin-)ester, 70 mg in 1,7 ml n-Porpanol eingespritzt. Hierzu wird eine Lösung (200 ml) zugegeben, die in 200 ml 0,5 g Harnstoff, 1 g Natrium-Taurodesoxycholat, 200 mg Natriumchlorid und 2,9 g TRIS enthält und deren pH-Wert auf 7,5 eingestellt ist, gegeben. Nach gutem Mischen werden 2,5 ml der so hergestellten Lösung mit 100 ul Probe (Serum) versetzt. Die Reaktion wird bei 578 nm Hg photometrisch verfolgt und wie in Beispiel 22 angegeben ausgewertet.

Testspezifische Kenngrößen (vgl. Beisp. 23 und Tabelle I):

| | |
|---|---|
| Leerwert: | 0,4 mE/min |
| Lipaseempfindlichkeit: | 28 mE/min pro 100 mE/min |
| Korrelationskoeffizient: | 0,988 |

**Beispiel 30**

4-Mehtylresorufin:

Herstellung wie im Patent DE 34 11 574 beschrieben aus 7,5 g 2-Methyl-4-nitrosoresorcin, 4,2 g Resorcin, 3,6 g Braunstein, 4,3 ml Schwefelsäure, 75 ml Methanol, 4 g Zinkpulver und 18 ml 25%igem Ammoniak.
DC: $R_f$ = 0,70 (Ethanol/Aceton 2:1)
UV/VIS (0,1M Kaliumphosphat-Puffer pH8,5): max = 579nm
Die Herstellung des 2-Methyl-nitrosoresorcins erfolgt gemäß DE 34 11 574, A 1.

**Beispiel 31**

1,2-O-Dilauryl-rac-glycero-3-glutarsäure-(4-methylumbelliferyl-)ester:

a) 2,75 g (5 mM) 7a und 1 g (5mM) 4-Methylumbelliferon werden in 30 ml Tetrahydrofuran gelöst und dann 2 g (10 mM) Dicyclohexylcarbodiimid und 0,15 g Dimethylaminopyridin zugefügt. Nach 24-stündigem Rühren bei Raumtemperatur wird filtriert und eingeengt. Das Rohprodukt wird durch Flash-Chromatographie gereinigt.
DC: $R_f$ = 0,40 (Essigester/Petrolether 1:3)
b) Die Emulsionsherstellung erfolgt wie in Beispiel 22 angegeben, es werden aber anstelle des dort eingesetzten Lipasesubstrats 35 mg 1,2-Dilauryl-rac-glycero-3-glutarsäure-(4-methylumbelliferyl)-ester in 1,7 ml n-Propanol gelöst, eingesetzt.

Die Reaktion wird fluorimetrisch bei 25 ° C verfolgt und zwar bei einer Anregungswellenlänge von 364 nm, Spaltbreite 5 nm und einer Emissionswellenlänge von 448 nm und einer Spaltbreite von 10 nm

Testspezifische Kenngrößen (vgl. Beispiel 23 und Tabelle I):

| | |
|---|---|
| Leerwert: | 0,4 mE/min |
| Lipaseempfindlichkeit: | 28mE/min pro 100 U/l |
| Korrelationskoeffizient: | 0,9942 |

**Beispiel 32**

1,2-O-Dilauryl-rac-glycero-3-glutarsäure-(p-nitrothiophenylester:

Herstellung von 32a - b) analog Beispielen 7a - b.

c) Herstellung analog Beispiel 1c) aus 1,4 g (10 mM) p-Nitrothiophenol, 100 ml DimethylFORMAMID, 2,4 ml Pyridin, 0,2 g Dimethylaminopyridin und 32 b.

DC: $R_f$ = 0,76 (Essigester/Hexan 1:4)

d) In 27 ml dest. Wasser werden die folgenden Verbindungen unter gutem Rühren gelöst:

| | |
|---|---|
| 4,83 mg | Natrium-Desoxycholat |
| 28,00 mg | CHES* |
| 17,50 g | Harnstoff |
| 2,13 mg | Natriumchlorid |
| 0,16 mg | Colipase vom Schwein |
| 0,33 mg | Calciumchlorid |
| 152,00 mg | Natrium-Taurodesoxycholat |

Der pH-Wert der Lösung wird auf pH = 8,3 eingestellt. Zu dieser Lösung wird unter gutem Rühren eine Lösung von 1,2-O-Dilauryl-rac-glycero-3-glutarsäure-(p-nitrothiophenyl)-estereingespritzt. Anschließend wurde die Lösung unter Kühlen 2 min mit Ultraschall behandelt (mittlere Intensität).

Zu 1 ml der so erhaltenen Emulsionslösung wurden 50 ul Probe (Lipasehaltiges Humanserum) gegeben und vermischt. Die Reaktion wurde bei 405 nm Hg photometrisch verfolgt und wie in Beispiel 22 angegeben berechnet.

testspezifische Kenngrößen:

| | |
|---|---|
| Leerwert: | 0,3 mE/min |
| Lipaseempfindlichkeit: | 12,2 mE/min/pro 100 U/L |
| Korrelationskoeffizient: | 0,998 |

**Beispiel 33**

a) 1-O-(2-Methoxy-octadecyl)-2-O-methyl-rac-glycero-3-glutarsäure-monoester:

Herstellung analog Beispiel 1a) aus 3g (7,7 mM) 1-O-(2-Methoxy-octadecyl)-2-O-methyl-glycerin, 25 ml Chloroform, 1,8 ml Pyridin, 0,1 g Dimethylaminopyridin und 1,75 (15 mM) Glutarsäureanhydrid.

Ausbeute: 1,5 g (39 %)

DC: $R_f$ = 0,68 (RP18, Aceton/Ethanol 1:2)

1-O-(2-Methoxy-octadecyl)-2-O-methyl-rac-glycero-3-glutarsäure-(6-methylresorufin)-ester:

b) Herstellung analog Beispiel 1b) aus 1,5 g (3 mM) 33a) und 1,5 ml Oxalylchlorid

c) Herstellung analog Beispiel 2c) aus 0,68 g (3 mM) 4-Methylresorufin, 0,45 ml 1,8-Diazabicyclo-(5,4,0)undec-7en und 33b). DC: $R_f$ = 0,86 (Essigester)

d) Die Herstellung der Testemulsion erfolgt wie im Beispiel 22 angegeben, jedoch werden diesmal als Lipasesubstrat 1-0-(2-Methoxy-octadecyl)-2-0-methyl-rac-glycero-3-glutarsäure-(6-methyl-resorufin)-ester eingesetzt.

Die Ermittlung der Lipasekonzentration erfolgt über eine Eichgerade aus zwei Standards unter schiedlicher Lipasekonzentration, bei der die Extinktionsänderung pro Minute gegen die KONZENTRATION aufgetragen ist.

| | |
|---|---|
| Leerwert: | 8,4 mE/min |
| Esteraseempfindlichkeit: | 25,3 mE/min |
| Lipaseempflindlichkeit: | 19,0 mE/min pro 100 U/l |
| Korrelationskoeffiezient: | 0,9483 |

**Beispiel 34**

CHES* = 2-(Cyclohexylamino)-aethan-sulfonsäure

a) 1,2-0-Dilauryl-rac-glycero-3-bernsteinsäure-monoester:
Herstellung analog Beispiel 1a) aus 8,56 g (20 mM) 1,2-0-Dilaurylglycerin, 4 g (40 mM) Bernsteinsäureanhydrid, 60 ml Chloroform, 4,6 ml Pyridin und 0,24 g Dimethylaminopyridin. Das Produkt kristallisiert aus Hexan.
Fp: 41 - 43 °C
DC: $R_f$ = 0,26 (Essigester/Hexan 1:4)
1,2-0-Dilauryl-rac-glycero-3-bernsteinsäure-resorufin-ester:
b) Herstellung analog Beispiel 17) aus 2,65 g (5 mM) 34a) 1,06 g (5 mM) Resorufin, 6,18 g (30 mM) Dicyclohexylcarbodiimid, 0,1 g Dimethylaminopyridin und 50 ml Dimethylformamid.
DC: $R_f$ = 0,47 (Essigester/Petrolether 1:3)
c) Wie in Beispiel 22 angegeben wird auch hier die Emulsion hergestellt. Als Lipasesubstrat werden in diesem Falle aber 70 mg 1,2-0-Dilauryl-rac-glycero-3-bernsteinsäure-resorufinesterin 1,7 ml Propanol gelöst, verwendet.

Dabei wurden die folgenden testspezifischen Kenngrößen erhalten(vgl. Beispiel 23 und Tabelle I):

| | |
|---|---|
| Leerwert: | 3,8 - 4,0 mE/min |
| Esteraseempfindlichkeit: | nicht ermittelt |
| Lipaseempfindlichkeit: | 8,7 mE/min pro 100 U/l |
| Korrelationskoeffizient: | 0,8793 |

**Beispiel 35**

a) 2-0-Lauryl-octadecandiol(1,2)-1-glutarsäure-monoester:
Herstellung analog Beispiel 1a) aus 4,6 g (10 mM) 2-0-Lauryl-octadecandiol(1,2), 29 ml Chloroform 2,3 ml Pyridin, 0,12 g Dimethylaminopyridin, 2,3 g (20 mM) Glutarsäureanhydrid
DC: $R_f$ = 0,54 (Petrolether/ Essigester 4:1)
2-0-Lauryl-octadecandiol(1,2)-1-glutarsäure-(6-methylresorufin-)ester:
b) Herstellung analog Beispiel 1b) aus 1,15 g (2mM) 35a) und 0,88 ml Oxalylchlorid
c) Herstellung analog Beispiel 2c) aus 0,45 g (2 mM) 4-Methylresorufin, 20 ml Chloroform, 0,3 ml 1,8-Diazabicyclo-(5,4,0)-undec-7-en, 40 mg Dimethylaminopyridin und 35b).
DC: $R_f$ = 0,37 (Petrolether/Essigester 5:1)
d) Wie in Beispiel 22 angegeben wird eine Emulsion hergestellt, jedoch werden als Lipasesubstrat 70 mg 2-0-Lauryl-octadecandiol-1.2-1-glutarsäure-(6-methylresorufin)-ester in 1,7 ml n-Propanol gelöst, eingesetzt.

Entsprechend dem Beispiel 23, Tab.I wurden die folgenden testspezifischen Kenngrößen erhalten:

| | |
|---|---|
| Leerwert: | 0,3 - 1,0 mE/min |
| Esteraseempfindlichkeit: | 1,0 mE/min |
| Lipaseempfindlichkeit: | 25,5 mE/min pro 100 U/l |
| Korrelationskoeffizient: | 0,996 |

**Beispiel 36**

a) 1,2-0-Dilauryl-rac-3-thioglycero-3-S-glutarsäuremonoester:
Herstellung analog Beispiel 1a) aus 2,5 g (4,8 mM) 1,2-0-Dilauryl-rac-3-thioglycerin, 14 ml Chloroform, 1,1 ml Pyridin und 1,1 g (9,6 mM) Glutarsäureanhydrid.
DC: $R_f$ = 0,5 (Hexan/Tetrahydrofuran 1:4)
1,2-0-Dilauryl-rac-3-thioglycero-3-S-glutarsäure-(6-methylresorufin-)ester:
b) Herstellung analog Beispiel 1b) aus 0,74 g (1,3 mM) 36a) und 0,6 ml Oxalylchlorid.
c) Herstellung analog Beispiel 2c) aus 0,3 g (1,3 mM) 4-Methylresorufin, 13 ml Chloroform, 0,2 ml 1,8-Diazabicyclo-(5,4,0)-undec-7-en, 27 mg Dimethylaminopyridin und 36b). DC: $R_f$ = 0,38 (Petrolether/Essigester 17:3)
Das als Ausgangsmaterial eingesetzte 1,2-0-Dilauryl-rac-3-thioglycerin wird analog Org. Synth III, S. 366 und S. 363 folgendermaßen erhalten:
Umsetzung von 1,2-0-Dilaurylglycerin mit Toluolsulfonsäurechlorid zum Dilauryl-glycero-3-toluolsulfonat, dann Reaktion mit Thioharnstoff zum entsprechenden Isothiuroniumsalz, anschließende Hydrolyse mit Salzsäure..
DC $R_f$ = 0,52 ( Petrolether/Essigester 49:1)
d) Wie in Beispiel 22 angegeben wird eine Emulsion hergestellt. Es wird jedoch anstelle des dort verwendeten Lipasesubstrats eine Lösung von 70 mg 1,2-0-Dilauryl-rac-3-thioglycero-3-S-glutarsäure-(6-methylresorufin)-ester in 1,7 ml n-Propanol eingesetzt.
testspezifische Kenngrößen:

| | |
|---|---|
| Leerwert: | 0,2 -0,9 mE/min |
| Esteraseempflindlichkeit: | 1,3 mE/min |
| Lipaseempfindlichkeit: | 4,1 mE/min pro 100 U/l |
| Korrelationskoeffizient: | 0,857 |

**Beispiel 37**

a) 1,2-S-Dilauryl-rac-1,2-dithioglycero-3-glutarsäuremonoester:
Herstellung analog Beispiel 1a) aus 3 g (6,5 mM) 1,2-S-Dilauryl-1,2-dithioglycerin, 30 ml Pyrdin und 1,5 g (13 mM) Glutarsäureanhydrid.
DC: $R_f$ = 0,43 (Petrolether/Essigester 1:1)
1,2-S-Dilauryl-rac-1,2-dithioglycero-3-glutarsäure-(6-methyl-resorufin-)ester:
b) Herstellung analog Beispiel 1b) aus 1,2 g (2 mM) 37a) und 1 ml Oxalylchlorid
c) Herstellung analog Belspie 2c) aus 0,46 g (2 mM) 4-Methylresorufin, 20 ml Chloroform, 0,3 ml 1,8-Diazabicyclo-(5,4,0)-undec-7-en und 37b)
DC: $R_f$ = 0,37 (Essigsäure/Petrolether 1 : 4)
Das als Ausgangsmaterial eingesetzte 1,2-S-Dilauryl-rac-1,2-dithioglycerin wird folgendermaßen hergestellt:
Zu einer Lösung von 9 g (160 mM) Kaliumhydroxyd in 250 ml Ethanol wird bei Raumtemperatur 10g (80 mM) 2,3-Dimercaptopropanol in 100 ml Ethanol getropft. Nach einstündigem Rühren wird eine Lösung von 40 g (160 mM) Dodecylbromid in 100 ml Ethanol zugetropft. Zur Vervollständigung der Reaktion wird noch zwei Tage gerührt, dann filtriert und das Filtrat mit Eis versetzt. Nach Ansäuern mit 2 N Salzsäure wird dreimal mit Ether extrahiert, die organische Phase getrocknet und eingeengt. Der Rückstand wird über eine Kieselgelsäule (Elutionsmittel: Essigester/Petrolether 1 : 10) gereinigt. DC $R_f$ = 0,54 (Essigester/Petrolether 1:10)
d) Wie in Beispiel 22 angegeben wird eine Emulsion hergestellt. Anstelle des dort eingesetzten Lipasesubstrats wird in diesem Falle aber 1.2-S-Dilauryl-rac-1,2-dithioglycero-3-S-glutar-säure-(6-methylresorufin-)ester, 70 mg in 1,7 ml n-Popranol gelöst, eingesetzt.

| | |
|---|---|
| Leerwert : | 3 mE/min. |
| Esteraseempfindlichkeit: | 3,4 mE/min. |
| Lipaseempfindlichkeit : | 24 mE/min. pro 100 U/l |
| Korrelationskoeffizient: | 0,9943 |

**Patentansprüche**
**Patentansprüche für folgende Vertragsstaaten : BE, CH, DE, FR, GB, IT, LI, LU, NL, SE**

**1.** Verbindung der allgemeinen Formel

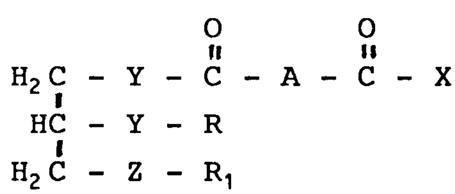

worin
A eine Alkylen- oder Alkenylengruppe mit 1 bis 16 C-Atomen,
R und $R_1$, die gleich oder verschieden sein können, je eine Alkyl-, Alkenyl- oder Acylgruppe mit 1 bis 20 C-Atomen oder eine gegebenenfalls alkylsubstituierte Aryl- oder Aralkylgruppe mit 1 bis 8 C-Atomen in Alkylrest, einer der Reste R und $R_1$ auch ein Wasserstoffatom, X den Rest einer aromatischen Hydroxy- oder Thiolverbindung, die einen Chromophor darstellt oder erst durch eine Folgereaktion in einen Farbstoff überführt werden kann, und jedes Y und Z unabhängig voneinander -S- oder -O-, Z auch $-CH_2-$, bedeuten.

**2.** Verbindung nach Anspruch 1,

**dadurch gekennzeichnet**,
daß R oder/und $R_1$ 8 bis 12 C-Atome aufweist.

**3.** Verbindung nach Anspruch 1 oder 2,
**dadurch gekennzeichnet**,
daß A 3 bis 7 C-Atome aufweist.

**4.** Verbindung nach Anspruch 1, 2 oder 3,
**dadurch gekennzeichnet**,
daß X ein gegebenenfalls substituierter Resorufin-, ein Chlorphenolrot-, Indoxyl-, Naphthol-, Thiophenol-, Thiofluoreszein- oder Phenolrest ist.

**5.** 1,2-O-Dioctyl-rac-glycero-3-azelainsäure-resorufinester.

**6.** 1,2-O-Didecyl-rac-glycero-3-pimelinsäure-resorufinester.

**7.** 1,2-O-Didecyl-rac-glycero-3-glutarsäure-resorufinester.

**8.** 1,2-O-Didodecyl-rac-glycero-3-glutarsäure-resorufinester.

**9.** Verfahren zur optischen Bestimmung der Lipase,
**dadurch gekennzeichnet**,
daß man ein Substrat gemäß Anspruch 1 der Einwirkung der lipasehaltigen Probe unterwirft und die Menge der freigesetzten aromatischen Hydroxy- oder Thiolverbindung direkt oder, nach Kopplung mit einem geeigneten Chromogen die daraus gebildete Farbe, optisch bestimmt.

**10.** Verfahren nach Anspruch 9, **dadurch gekennzeichnet**, daß man die Bestimmung bei einer Salzkonzentration im Test von 0,1 bis 10 mg/ml vornimmt.

**11.** Reagenz zur optischen Bestimmung der Lipase,
**dadurch gekennzeichnet**,
daß es wenigstens eine Verbindung nach einem der Ansprüche 1 bis 8 und Puffersubstanz, sowie Gallensäurealyalisalz, Colipase, Salz, Harnstoff oder/und einen chromogenen Kuppler enthält.

**12.** Reagenz nach Anspruch 11,
**dadurch gekennzeichnet**,
daß es
0,05 bis 10 mg/ml Substrat,
2 bis 50 mg/ml Desoxycholat,
0,001 bis 0,01 mg/ml Colipase,
1 bis 100 mg/ml Harnstoff,
0,1 bis 10 mg/ml NaCl,
1 bis 50 mg/ml Puffersubstanz,
jeweils bezogen auf gebrauchsfertige Lösung im Test, enthält.

**13.** Reagenz nach Anspruch 11 oder 12,
**dadurch gekennzeichnet**,
daß es auf einem Trägermaterial imprägniert vorliegt.

**Patentansprüche für folgenden Vertragsstaat : AT**

**1.** Verfahren zur Herstellung einer Verbindung der allgemeinen Formel

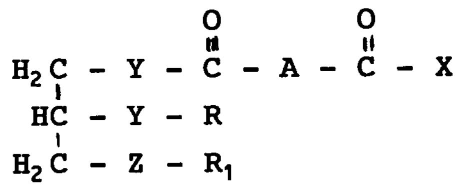

worin
A eine Alkylen- oder Alkenylengruppe mit 1 bis 16 C-Atomen,
R und $R_1$, die gleich oder verschieden sein können, je eine Alkyl-, Alkenyl- oder Acylgruppe mit 1 bis 20 C-Atomen oder eine gegebenenfalls alkylsubstituierte Aryl- oder Aralkylgruppe mit 1 bis 8 C-Atomen in Alkylrest, einer der Reste R und $R_1$ auch ein Wasserstoffatom, X den Rest einer aromatischen Hydroxy- oder Thiolverbindung, die einen Chromophor darstellt oder erst durch eine Folgereaktion in einen Farbstoff überführt werden kann, und jedes Y und Z unabhängig voneinander -S- oder -O-, Z auch $-CH_2-$, bedeuten,
**dadurch gekennzeichnet,**
daß man aus den entsprechenden 1.2.-O-Diether- und 1.2-Diacyl-glycerinverbindungen mit den entsprechenden Dicarbonsäureanhydriden in wasserfreiem Medium wie Chloroform/Pyridin zu dem entsprechenden Glycerodicarbonsäure-Monoester umsetzt und letzteren mit der aromatischen Hydroxy- oder Thiolverbindung, von der sich der Rest X ableitet, verestert oder in einen aktivierten Ester überführt, beispielsweise in den Hydroxysuccinimidester oder das Imidazolid und den aktivierten Ester dann mit dem aromatischen Alkohol oder Thiol umsetzt.

**2.** Verfahren nach Anspruch 1,
**dadurch gekennzeichnet**,
daß R oder/und $R_1$ 8 bis 12 C-Atome aufweist.

**3.** Verfahren nach Anspruch 1 oder 2,
**dadurch gekennzeichnet**,
daß A 3 bis 7 C-Atome aufweist.

**4.** Verfahren nach Anspruch 1, 2 oder 3,
**dadurch gekennzeichnet**,
daß X ein gegebenenfalls substituierter Resorufin-, ein Chlorphenolrot-, Indoxyl, Naphthol-, Thiophenol-, Thiofluoreszein- oder Phenolrest ist.

**5.** Verfahren zur optischen Bestimmung der Lipase,
**dadurch gekennzeichnet**,
daß man eine Verbindung der allgemeinen Formel von Anspruch 1 der Einwirkung der lipasehaltigen Probe unterwirft und die Menge der freigesetzten aromatischen Hydroxy- oder Thiolverbindung direkt oder, nach Kopplung mit einem geeigneten Chromogen die daraus gebildete Farbe, optisch bestimmt.

**6.** Verfahren nach Anspruch 5,
**dadurch gekennzeichnet**,
daß man die Bestimmung bei einer Salzkonzentration im Test von 0,1 bis 10 mg/ml vornimmt.

**7.** Reagenz zur optischen Bestimmung der Lipase,
**dadurch gekennzeichnet,**
daß es wenigstens eine Verbindung der allgemeinen Formel von Anspruch 1 und Puffersubstanz, sowie Gallensäurealkalisalz, Colipase, Salz, Harnstoff oder/und eine chromogenen Kuppler enthält.

**8.** Reagenz nach Anspruch 7,
**dadurch gekennzeichnet**,
daß es
0,05 bis 10 mg/ml Substrat der allgemeinen Formel von Anspruch 1,
2 bis 50 mg/ml Desoxycholat,
0,001 bis 0,01 mg/ml Colipase,

1 bis 100 mg/ml Harnstoff,
0,1 bis 10 mg/ml NaCl,
1 bis 50 mg/ml Puffersubstanz,
jeweils bezogen auf gebrauchsfertige Lösung im Test, enthält.

**9.** Reagenz nach Anspruch 7 oder 8,
**dadurch gekennzeichnet**,
daß es auf einem Trägermaterial imprägniert vorliegt.

## Claims

**Claims for the following Contracting States : BE, CH, LI, DE, FR, GB, IT, LU, NL, SE**

**1.** Compound of the general formula:-

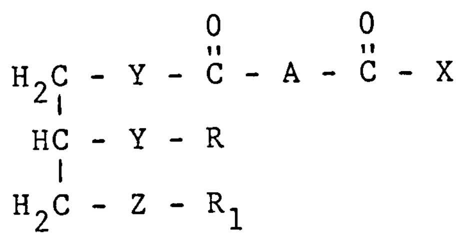

wherein A signifies an alkylene or alkenylene group with 1 to 16 C-atoms, R and $R_1$, which can be the same or different, each signify an alkyl, alkenyl or acyl group with 1 to 20 C-atoms or a possibly alkyl-substituted aryl or aralkyl group with 1 to 8 C-atoms in the alkyl radical, one of the radicals R and $R_1$ also a hydrogen atom, X the residue of an aromatic hydroxy or thiol compound, which represents a chromophore or can first be converted into a coloured material by a subsequent reaction, and each Y and Z, independently from each other, is -S- or -0-, Z also $-CH_2-$.

**2.** Compound according to claim 1, characterised in that R and/or $R_1$ have 8 to 12 C-atoms.

**3.** Compound according to claim 1 or 2, characterised in that A has 3 to 7 C-atoms.

**4.** Compound according to claim 1, 2 or 3, characterised in that X is a possibly substituted resorufin, a chlorophenol red, indoxyl, naphthol, thiophenyl, thiofluorescein or phenol radical.

**5.** 1,2-0-Dioctyl-rac-glycero-3-azelaic acid resorufin ester.

**6.** 1,2-0-Didecyl-rac-glycero-3-pimelic acid resorufin ester.

**7.** 1,2-0-Didecyl-rac-glycero-3-glutaric acid resorufin ester.

**8.** 1,2-0-Didodecyl-rac-glycero-3-glutaric acid resorufin ester.

**9.** Process for the optical determination of lipase, characterised in that one subjects a substrate according to claim 1 to the action of the lipase-containing sample and determines the amount of liberated aromatic hydroxy or thiol compound optically directly or, after coupling with a suitable chromogen, the colour formed therefrom.

**10.** Process according to claim 9, characterised in that one carries out the determination at a salt concentration in the test of 0.1 to 10 mg/ml.

**11.** Reagent for the optical determination of lipase, characterised in that it contains at least one compound according to one of claims 1 to 8 and buffer substance, as well as bile acid alkali metal salt, colipase, salt, urea and/or a chromogenic coupler.

**12.** Reagent according to claim 11, characterised in that it contains:
0.05 to 10 mg/ml of substrate,
2 to 50 mg/ml desoxycholate,
0.001 to 0.01 mg/ml colipase,
1 to 100 mg/ml urea,
0.1 to 10 mg/ml NaCl
1 to 50 mg/ml buffer substance,
in each case referred to the solution ready for use in a test.

**13.** Reagent according to claim 11 or 12, characterised in that it is present impregnated on a carrier material.

**Claims for the following Contracting State : AT**

**1.** Process for the preparation of a compound of the general formula:-

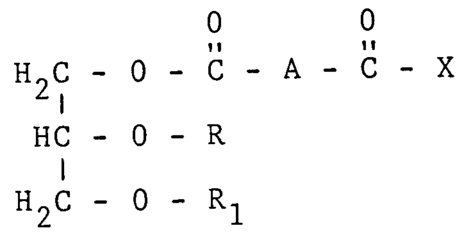

wherein A signifies an alkylene or alkenylene group with 1 to 16 C-atoms, R and $R_1$, which can be the same or different, each signify an alkyl, alkenyl or acyl group with 1 to 20 C-atoms or a possibly alkyl-substituted aryl or aralkyl group with 1 to 8 C-atoms in the alkyl radical, one of R and $R_1$ also a hydrogen atom, X the residue of an aromatic hydroxy or thiol compound, which represents a chromophore or can first be converted into a coloured material by a subsequent reaction, characterised in that one reacts the corresponding 1,2-0-diether- and 1,2-diacyl-glycerol compounds with the corresponding dicarboxylic acid anhydrides in anhydrous medium, such as chloroform/pyridine, to give the corresponding glycerodicarboxylic acid monoester and esterifies the latter with the aromatic hydroxy or thiol compound from which the radical X is derived or converts into an activated ester, for example into the hydroxysuccinimide ester or the imidazolide, and then reacts the activated ester with the aromatic alcohol or thiol.

**2.** Process according to claim 1, characterised in that R and/or $R_1$ has 8 to 12 C-atoms.

**3.** Process according to claim 1 or 2, characterised in that A has 3 to 7 C-atoms.

**4.** Process according to claim 1, 2 or 3, characterised in that X is a possibly substituted resorufin, a chlorophenol red, indoxyl, naphthol, thiophenol, thiofluorescein or phenol radical.

**5.** Process for the optical determination of lipase, characterised in that one subjects a compound of the general formula of claim 1 to the action of the lipase-containing sample and determines the amount of liberated aromatic hydroxy or thiol compound optically directly or, after coupling with a suitable chromogen, the colour formed therefrom.

**6.** Process according to claim 5, characterised in that one carries out the determination at a salt concentration in the test of 0.1 to 10 mg/ml.

**7.** Reagent for the optical determination of lipase, characterised in that it contains at least one compound of the general formula of claim 1 and buffer substance, as well as bile acid alkali metal salt, colipase, salt, urea and/or a chromogenic coupler.

**8.** Reagent according to claim 7, characterised in that it contains:

0.05 to 10 mg/ml substrate of the general formula of claim 1,
2 to 50 mg/ml desoxycholate,
0.001 to 0.01 mg/ml colipase,
1 to 100 mg/ml urea,
0.1 to 10 mg/ml NaCl
1 to 50 mg/ml buffer substance,
in each case referred to the solution ready for use in a test.

**9.** Reagent according to claim 7 or 8, characterised in that it is present impregnated on a carrier material.

**Revendications**
**Revendications pour les Etats contractants suivants : BE, CH, LI, DE, FR, GB, IT, LU, NL, SE**

**1.** Composé de formule générale

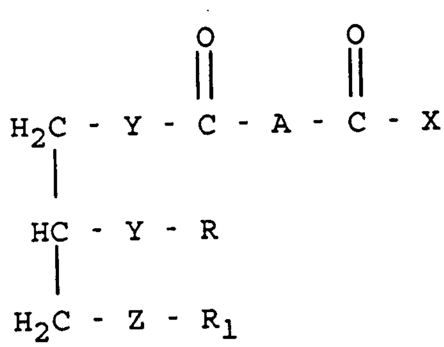

dans laquelle
A représente un groupe alkylène ou alcénylène de 1 à 16 atomes de carbone,
R et $R_1$, qui peuvent être identiques ou différents, représentent chacun un groupe alkyle, alcényle ou acyle de 1 à 20 atomes de carbone ou un groupe aryle ou aralkyle éventuellement alkylsubstitué de 1 à 8 atomes de carbone dans le reste alkyle, l'un des restes R et $R_1$ représentant aussi un atome d'hydrogène, X représente le reste d'un composé hydroxy ou thiol aromatique qui représente un chromophore ou qui peut n'être converti en un colorant que par une réaction consécutive, et chaque Y et Z représentent indépendamment l'un de l'autre -S- ou -O-, z représentant aussi $-CH_2-$.

**2.** Composé selon la revendication 1, caractérisé en ce que R et/ou $R_1$ présente 8 à 12 atomes de carbone.

**3.** Composé selon la revendication 1 ou 2, caractérisé en ce que A présente 3 à 7 atomes de carbone.

**4.** Composé selon la revendication 1, 2 ou 3, caractérisé en ce que X est un reste résorufine éventuellement substitué, un reste rouge de chlorophénol, indoxyle, naphtole, thiophénol, thiofluorescéine ou phénol.

**5.** 1,2-O-dioctyl-rac-glycéro-3-azélate de résorufine.

**6.** 1,2-O-didécyl-rac-glycéro-3-pimélate de résorufine.

**7.** 1,2-O-didécyl-rac-glycéro-3-glutarate de résorufine.

**8.** 1,2-O-didodécyl-rac-glycéro-3-glutarate de résorufine.

**9.** Procédé de détermination optique de la lipase, caractérisé en ce que l'on soumet un substrat selon la revendication 1 à l'action de l'échantillon contenant la lipase et l'on détermine optiquement directement la quantité de composé hydroxy ou thiol aromatique libéré, ou bien l'on détermine optiquement après couplage avec un chromogène la couleur ainsi formée.

**10.** Procédé selon la revendication 9, caractérisé en ce que l'on accomplit la détermination à une concentration de sel dans l'essai de 0,1 à 10 mg/ml.

**11.** Réactif pour la détermination optique de la lipase, caractérisé en ce qu'il contient au moins un composé selon l'une des revendications 1 à 8 et une substance tampon ainsi qu'un sel alcalin de l'acide biliaire, la colipase, du sel, de l'urée et/ou un coupleur chromogène.

**12.** Réactif selon la revendication 11, caractérisé en ce qu'il contient
0,05 à 10 mg/ml de substrat,
2 à 50 mg/ml de désoxycholate,
0,001 à 0,01 mg/ml de colipase,
1 à 100 mg/ml d'urée,
0,1 à 10 mg/ml de NaCl,
1 à 50 mg/ml de substance tampon,
à chaque fois par rapport à la solution prête à l'emploi dans l'essai.

**13.** Réactif selon la revendication 11 ou 12, caractérisé en ce qu'il impreigne un matériau support.

**Revendications pour l'Etat contractant suivant : AT**

**1.** Procédé de préparation d'un composé de formule générale

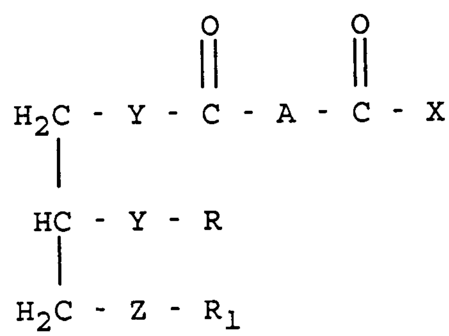

dans laquelle
A représente un groupe alkylène ou alcénylène de 1 à 16 atomes de carbone,
R et $R_1$, qui peuvent être identiques ou différents, représentent chacun un groupe alkyle, alcényle ou acyle de 1 à 20 atomes de carbone ou un groupe aryle ou aralkyle éventuellement alkylsubstitué de 1 à 8 atomes de carbone dans le reste alkyle, l'un des restes R et $R_1$ représentant aussi un atome d'hydrogène, X représente le reste d'un composé hydroxy ou thiol aromatique qui représente un chromophore ou qui peut n'être converti en un colorant que par une réaction consécutive, et chaque Y et Z représentent indépendamment l'un de l'autre -S- ou -O-, z représentant aussi $-CH_2-$,
caractérisé en ce que l'on réalise à partir des composés de 1.2.-O-diéther- et 1.2-diacylglycérine correspondants et des anhydrides de diacides carboxyliques correspondants la conversion en le monoester glycérodicarboxylique dans un milieu anhydre tel que chloroforme/pyridine et on estérifie ce dernier avec le composé hydroxy ou thiol aromatique duquel provient le reste X, ou bien on le convertit en un ester activé, par exemple en l'ester d'hydroxysuccinimide ou en l'imidazolide et on fait ensuite réagir l'ester activé avec l'alcool ou thiol aromatique.

**2.** Procédé selon la revendication 1, caractérisé en ce que R et/ou $R_1$ présente 8 à 12 atomes de carbone.

**3.** Procédé selon la revendication 1 ou 2, caractérisé en ce que A présente 3 à 7 atomes de carbone.

**4.** Procédé selon la revendication 1, 2 ou 3, caractérisé en ce que X est un reste résorufine éventuellement substitué, un reste rouge de chlorophénol, indoxyle, naphtole, thiophénol, thiofluorescéine ou phénol.

5. Procédé de détermination optique de la lipase, caractérisé en ce que l'on soumet un composé de formule générale selon la revendication 1 à l'action de l'échantillon contenant la lipase et l'on détermine optiquement directement la quantité de composé hydroxy ou thiol aromatique libéré, ou bien l'on détermine optiquement après couplage avec un chromogène la couleur ainsi formée.

6. Procédé selon la revendication 5, caractérisé en ce que l'on accomplit la détermination à une concentration de sel dans l'essai de 0,1 à 10 mg/ml.

7. Réactif pour la détermination optique de la lipase, caractérisé en ce qu'il contient au moins un composé de formule générale selon la revendication 1 et une substance tampon ainsi qu'un sel alcalin de l'acide biliaire, la colipase, du sel, de l'urée et/ou un coupleur chromogène.

8. Réactif selon la revendication 7, caractérisé en ce qu'il contient
0,05 à 10 mg/ml de substrat de formule générale selon la revendication 1,
2 à 50 mg/ml de désoxycholate,
0,001 à 0,01 mg/ml de colipase,
1 à 100 mg/ml d'urée,
0,1 à 10 mg/ml de NaCl,
1 à 50 mg/ml de substance tampon,
à chaque fois par rapport à la solution prête à l'emploi dans l'essai.

9. Réactif selon la revendication 7 ou 8, caractérisé en ce qu'il imprègne un matériau support.